# DEMANDE DE BREVET EUROPEEN

(11) **EP 2 604 317 A1**
(43) Date de publication de la demande: **19.06.2013**
(21) Numéro de dépôt: 12197059.4
(22) Date de dépôt: 13.12.2012
(51) Int. Cl.: A61Q 19/06, A61K 8/97, A61K 8/64, A61K 8/34, A61K 8/06, A61K 36/76, A61K 36/62

(54) **Composition topique à visée amincissante**

(30) Priorité: 15.12.2011 FR 1161730
(71) Demandeur: CASTER, 75008 Paris (FR)
(72) Inventeur: Choulot, Jean-Christophe, 78120 Rambouillet (FR)
(74) Mandataire: Dossmann, Gérard

(57) **Abrégé**

La présente invention a pour objet une composition cosmétique contenant au moins un extrait de saule, un extrait de lotus et un peptide de séquence SEQ N°1. L'invention a également pour objet un procédé cosmétique pour lutter contre la cellulite et/ou la peau d'orange, comprenant l'application topique de la composition sur la peau.

## Description

La présente invention a pour objet une composition cosmétique contenant au moins un extrait de saule, un extrait de lotus et un peptide de séquence SEQ N°1. L'invention a également pour objet un procédé cosmétique pour lutter contre la cellulite et/ou la peau d'orange, comprenant l'application topique de la composition sur la peau.

Le tissu adipeux est un organe endocrine majoritaire qui exerce une influence profonde sur toute l'homéostasie corporelle. Deux types de tissus adipeux existent chez les mammifères, le tissu adipeux blanc et le tissu adipeux brun. Le tissu adipeux blanc est la réserve majoritaire d'énergie tandis que le tissu adipeux brun qui exprime la protéine UCP1 (uncoupling protein 1) permet la dissipation de l'énergie par l'intermédiaire de thermogénèse d'adaptation. L'excès de tissu adipeux blanc est la cause de l'accumulation de cellulite pouvant conduire à l'obésité.

La présence de tissu adipeux brun est bien connue chez de nombreux mammifères. Par exemple, chez les mammifères qui hibernent, le tissu adipeux brun s'active à la fin de l'hiver et commence à brûler des acides gras. Cette «combustion» produit de l'énergie sous forme de chaleur, grâce à laquelle l'animal recouvre peu à peu une température normale.

Ce tissu adipeux «thermogénique» est également présent chez beaucoup de petits mammifères non hibernants, en particulier les rongeurs chez lesquels il favorise leur adaptation au froid et est capable d'interférer avec l'obésité. Chez les grands mammifères, en revanche, il n'est présent que chez les nouveau-nés, auxquels il offre une protection contre l'hypothermie.

On a longtemps pensé que l'homme à l'âge adulte était lui aussi dépourvu de tissu adipeux brun. Cependant, dès les années 2000, les radiologues spécialisés en médecine nucléaire avaient admis l'hypothèse de la présence de graisse brune chez l'homme (T.F. Hany et al., Eur. J. Nucl. Med. Mol. Imaging, 29, 1393, 2002; H.W. Yeung et al., J. Nucl. Med., 44, 1789, 2003.

Il aura fallu attendre 2009 pour avoir la preuve qu'un tissu adipeux brun est présent chez l'adulte, au sein même du tissu adipeux blanc mais on connait peu de chose à propos de la présence et de la signification de cette graisse brune chez les humains (M.C. Zingaretti et al., (2009) FASEB J., 23, 3113 ; A.M. Cypess et al., (2009) N. Engl. J. Med., 360, 1509.

De plus, malgré les fonctions différentes des adipocytes bruns et blancs, les facteurs qui influencent la formation de cellules graisseuses blanches et brunes dans le corps sont inconnus. On considère cependant, qu'une quantité de 50 grammes de graisse brune stimulée pourrait suffire pour fournir jusqu'à 20 % des dépenses d'énergie journalière chez un être humain adulte (Rothwell, N.J., and Stock, M.J. Clin. Sci. (Lond.) 1983, vol 64, pp 19-23).

La graisse blanche et la graisse brune sont localisées dans des zones anatomiques distinctes chez les rongeurs bien qu'on puisse noter la présence d'adipocytes bruns dans des zones normalement dévolues à la graisse blanche dans certaines conditions. Les deux types de tissus graisseux diffèrent également ontogénétiquement dans les souris et les rats puisque la graisse blanche et la graisse brune se développent avant et après la natalité respectivement.

Chez les autres mammifères comme les humains, la graisse blanche et la graisse brune se développent avant la naissance. Plusieurs détails distinguent les adipocytes blancs des bruns. Les cellules de graisse blanche contiennent normalement une gouttelette majoritaire de lipide qui remplit la majorité du cytoplasme. Les cellules brunes contiennent plusieurs petites gouttelettes de lipide. Elles contiennent également beaucoup de fer, ce qui leur confère leur couleur brune.

Contrairement aux cellules de la graisse blanche, les adipocytes bruns contiennent une grande quantité de mitochondries mais au lieu de produire de l'énergie sous forme d'ATP (adénosine triphosphate), elles en dégagent sous forme de chaleur (thermogenèse).

Cette particularité confère aux adipocytes bruns présents dans la graisse blanche la capacité de détruire les adipocytes blancs lorsqu'il est nécessaire de produire de la chaleur. Ce mécanisme d'action est beaucoup plus rapide et performant que le déstockage des triglycérides des adipocytes blancs en acides gras qui peuvent être à nouveau stockés sous forme de graisse blanche. En effet, pour des raisons diverses (nourriture trop riche, inactivité, vieillissement et autres), il peut se produire dans les adipocytes de la graisse blanche une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant à la silhouette un aspect général disgracieux pouvant évoluer entre la simple surcharge locale et la formation de cellulite.

Ainsi, la graisse blanche et la graisse brune ont des fonctions pratiquement opposées *in vivo* puisque la graisse blanche accumule l'énergie en excès en tant que triacyl glycérol tandis que la graisse brune dissipe l'énergie par thermogénèse.

La capacité thermogénique de la graisse brune est due à l'expression d'UCP1 exclusivement présente dans les adipocytes bruns. L'UCP1 est un transporteur de protons facultatifs localisés dans la membrane interne mitochondriale dans lequel il peut générer de la chaleur. Chez les rongeurs, l'expression d'UCP1 s'effectue en réponse à des stimuli externes tels que les changements de température. Des souris transgéniques exprimant la toxine A diphtérique à partir du promoteur UCP1 présentent une réduction de 60 à 70% de la masse de la graisse brune et ces souris sont obèses et sensibles au froid. Globalement, chez la souris, la tendance à l'obésité est corrélée avec une décroissance de l'activité des cellules brunes.

De plus, les personnes maigres ont tendance à avoir plus de graisse brune. Ainsi, la quantité de tissu adipeux composé de cellules brunes est inversement proportionnelle à l'index de masse grasse, suggérant un rôle du tissu adipeux brun dans le métabolisme humain adulte. Il importe donc de stimuler la graisse brune afin de lutter contre l'adiposité et la cellulite, pouvant conduire à l'obésité.

Compte tenu de l'inconfort tant physique, esthétique que psychologique qu'elles occasionnent auprès des individus qui en sont atteints, en particulier les femmes, l'adiposité et la cellulite constituent en effet, de nos jours une affection de moins en moins bien supportée ou acceptée.

La demanderesse a mis au point de nouvelles compositions cosmétiques, qui constituent l'objet de l'invention.

L'invention a également pour objet un procédé cosmétique pour lutter contre la cellulite et/ou la peau d'orange, comprenant l'application sur la peau d'une composition telle que définie précédemment.

D'autres objets apparaîtront à la lecture de la description, des dessins et des exemples qui suivent.
Les figures 1 et 2 sont des observations photographiques de la morphologie des cellules effectuées sans (Fig 1) et après application de la composition contenant les actifs (Fig 2).

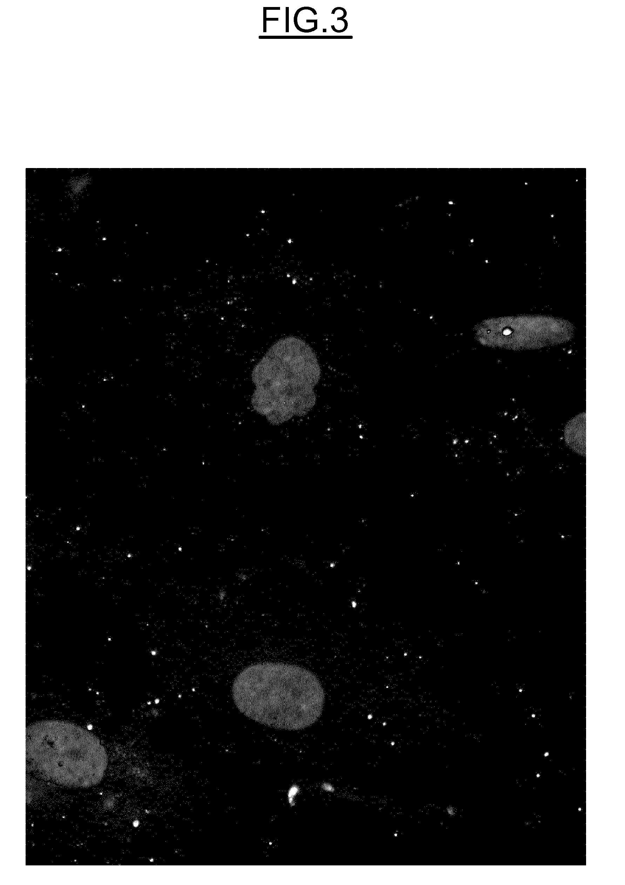

Les figures 3 et 4 sont des observations photographiques de la visualisation de l'expression de la protéine UCP1 par immunofluorescence effectuées sans (Fig 3) et après application de la composition contenant les actifs (Fig 4).

Les compositions conformes à l'invention comportent dans un milieu physiologiquement acceptable, au moins un extrait de saule, un extrait de lotus et un peptide de séquence SEQ N°1.

L'extrait de saule est un extrait de saule blanc, autrement appelé *Salix alba.* Cet extrait est obtenu de préférence par extraction à l'éthanol de l'écorce de ce végétal, suivie d'une filtration et de séchage pour obtenir l'extrait sec utilisé dans l'invention. Ainsi, de préférence, l'extrait de saule blanc est un extrait hydroalcoolique. Dans les compositions de l'invention, la concentration finale d'extrait sec de saule blanc est de préférence de 0.01 à 10% en poids de la composition totale.

L'extrait de lotus est obtenu par extraction à l'éthanol de la feuille de lotus *(Nelumbo nucifera)* suivie d'une filtration et de séchage pour obtenir un résidu sec. De préférence, l'extrait de lotus est un extrait hydroalcoolique. De préférence, dans les compositions de l'invention, la concentration finale d'extrait sec de lotus est de 0.01 à 10% en poids de la composition totale.

Le peptide utilisé dans les compositions de l'invention est un pentapeptide de séquence en acides aminés Lys-Val-Arg-Leu-Gln (SEQ ID N°1). Ce peptide de séquence similaire aux UCPs est vendu par la société Vinsciences (Sophia antipolis, France). Ce peptide de poids moléculaire de 642 Da est mis en solution dans un mélange eau/butylène glycol (60/40). Dans les compositions de l'invention, la concentration finale de peptide de séquence SEQ ID N°1 est de 0,0001 à 5% en poids de la composition totale.

La composition selon l'invention est adaptée à une application topique sur la peau et comprend donc généralement un milieu physiologiquement acceptable, c'est-à-dire compatible avec la peau. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut comprendre avantageusement une phase aqueuse. La composition peut comprendre de l'eau en une teneur allant de 1 à 99% en poids par rapport au poids total de la composition, de préférence allant de 40 à 75% en poids par rapport au poids total de la composition.

La composition selon l'invention peut se présenter sous toutes les formes galéniques normalement utilisées pour une application topique sur la peau, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick.

Avantageusement, la composition selon l'invention est sous forme d'une émulsion huile dans eau ou d'un gel, une crème ou un stick.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les co-émulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40 ; le tristéarate de sorbitane, les stéarates de sorbitane oxyéthylénés disponibles sous les dénominations commerciales Tween 20 ou Tween 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition définie précédemment est utilisée en application topique dans le cadre d'un procédé cosmétique pour lutter contre la cellulite et/ou la peau d'orange.

L'invention sera maintenant illustrée à l'aide des exemples non limitatifs suivants.

### Exemple 1 Différenciation des cellules

Des préadipocytes humains (Promocell, lot 5112901 : femme, caucasien, Index de Masse Grasse 21, 3^{ème} passage) sont mis en culture en plaques 24 puits à raison de 10 000 cellules par puits (Greiner) dans du milieu de croissance (Promocell, C-27410) puis placés en étuve à 37°C dans une atmosphère à 5% de CO₂. Le milieu de culture est renouvelé tous les 2-3 jours à raison de 1,5ml/puits.

Une fois la confluence des cellules atteinte, les préadipocytes sont ensuite différenciés en adipocytes humains en 10 jours environ dans un milieu de différenciation puis placés dans un milieu de nutrition pour adipocytes (Promocell C-24736 & C-27438). Brièvement, à J0, on débute la différenciation en plaçant les préadipocytes dans 1,5 mL de milieu de différenciation par puits. A J3, les cellules sont ensuite mises dans 1,5 mL de milieu de nutrition par puits. Après environ 10 jours, on vérifie en regardant la morphologie cellulaire au microscope : les cellules doivent être chargées en lipides.

### Exemple 2 : Mesure du taux de triglycérides intracellulaires lors de la différenciation

Cette mesure s'effectue à l'aide du test Adipored™ Assay Reagent (Lonza, PT-7009). Cette technique utilise la propriété du colorant hydrophile Nile Red, qui dispersé dans un environnement hydrophobe (gouttelettes lipidiques intracellulaires des adipocytes), devient fluorescent. On peut ainsi visualiser et quantifier la quantité de triglycérides marqués.

1,5 mL de réactif est ajouté dans les puits de la plaque 24 puits à confluence. Puis la fluorescence est mesurée (lecteur Safire, Tecan, λem=485-λex=572nm) et la production de triglycérides est suivie au cours du temps afin d'apprécier la cinétique de différenciation des préadipocytes en adipocytes.

On note une augmentation au cours du temps du signal de Fluorescence (RFU).

| | T₀ | T₅ⱼₒᵤᵣₛ | T₁₀ⱼₒᵤᵣₛ | T₁₄ⱼₒᵤᵣₛ |
|---|---|---|---|---|
| RFU | 845 | 4047 | 11338 | 10089 |

| | | | | |
|---|---|---|---|---|
| n=2 puits par mesure en points multiples sur chaque puits Le T₀ correspond au changement du milieu de croissance en milieu de différenciation (Promocell). | | | | |

On observe une augmentation du signal de fluorescence traduisant une production accrue de triglycérides pendant 10 jours ; puis le signal se stabilise à 10 jours et reste stable à 14 jours, signe de la différenciation complète des préadipocytes en adipocytes.

### Exemple 3 : Mesure du taux de triglycérides, de glycérol et de protéine UCP des adipocytes après traitement

Il s'agit de déterminer les effets de trois actifs, appliqués sur des adipocytes humains différenciés. Quatre paramètres sont analysés, la morphologie, le taux de triglycérides, la quantité de glycérol intracellulaire et l'expression de la protéine UCP-1 dans ces cellules après traitement.

Brièvement, après culture et traitement de 10 000 cellules dans des plaques 24 puits selon le protocole précédemment décrit, on dépose une composition contenant les actifs dilués dans du milieu de nutrition sur les cellules, soit 1.5 mL, 17 jours après le début de la différenciation (adipocytes « matures »).

Cette composition contient les actifs dans les quantités suivantes : un résidu sec de feuille de lotus résultant d'une extraction hydroglycolique (eau/éthanol à mélange égal) de *Nelumbo nucifera* filtrée et séchée, un extrait de saule blanc résultant d'une extraction hydroglycolique (eau/éthanol à mélange égal) de *Salix alba,* filtrée et séchée, et le Pentapeptide-25 (Vinscience UCPeptide™ V) de séquence Lys-Val-Arg-Leu-Gln en solution à 0.01% dans un mélange eau/butylène glycol (60/40).

Ces extraits secs sont dilués dans le milieu de culture de nutrition pour adipocytes (Promocell, C-27438). La concentration finale de ces extraits en solution est, en poids de la composition finale, de 0,5% pour la solution de lotus, de 0,6% pour la solution de saule blanc, et de 0,0001% de Pentapeptide-25.

Les milieux de culture sont laissés sur les cellules pendant 1 ou 3 jours après différenciation. Pendant cette étape de traitement, le milieu n'est pas renouvelé et le traitement n'est pas réappliqué.

### a) Mesure du taux de triglycérides intracellulaires

Cette mesure s'effectue sur les adipocytes humains préalablement différenciés, à l'aide du test Adipored™ Assay Reagent (Lonza, PT-7009) de la même manière que précédemment.

| RFU | J1 | J3 |
|---|---|---|
| Témoin | 10502 | 11352 |
| Apres traitement | 8399 | 5750 |

| | | |
|---|---|---|
| n=2 puits par mesure en points multiples sur chaque puits RFU : signal de Fluorescence | | |

La quantité de triglycérides dans les cellules diminue de près de 30 % après traitement des adipocytes par la composition de l'exemple 3 selon l'invention. Une inhibition de la synthèse des triglycérides est donc obtenue au bout de 72 h de traitement avec la composition de l'invention.

### b) Morphologie des cellules après traitement

Sur les adipocytes humains différenciés préparés au début de l'exemple 3, le colorant Oil Red O (Sigma 00625) permet d'apprécier l'accumulation des gouttelettes lipidiques en fonction du traitement. En effet ce colorant est utilisé pour visualiser les triglycérides neutres et les lipides.

On observe les cellules après 24 et 72 heures de traitement. A 72 heures, après fixation des cellules dans les puits de la plaque avec du formaldéhyde à 4 %, le marquage à l'huile rouge (Sigma Oil red 00625, France) diluée à 0,3% dans de l'isopropanol est effectué pendant 15 minutes puis les noyaux cellulaires sont contre colorés brièvement avec le réactif Hématoxylin de Mayer (Réactifs RAL, réf 361620-0125).

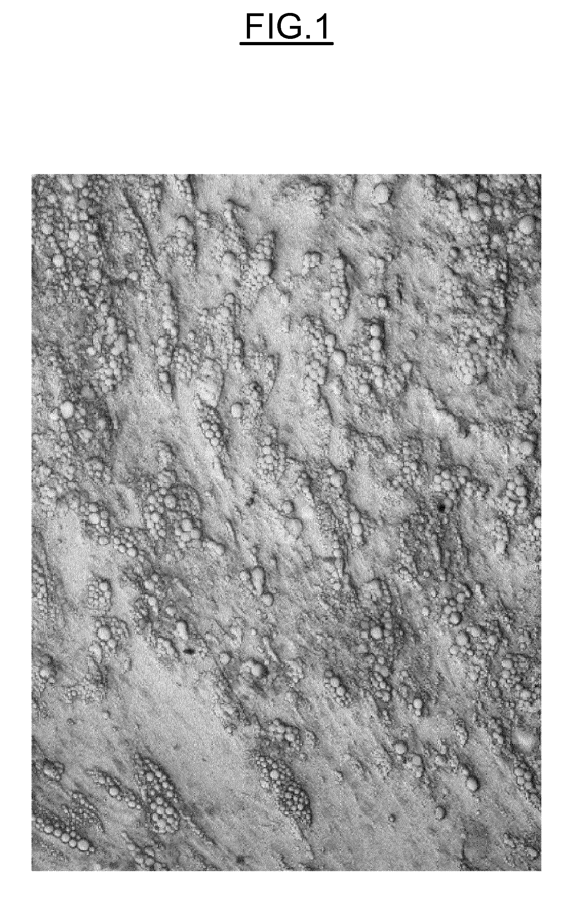

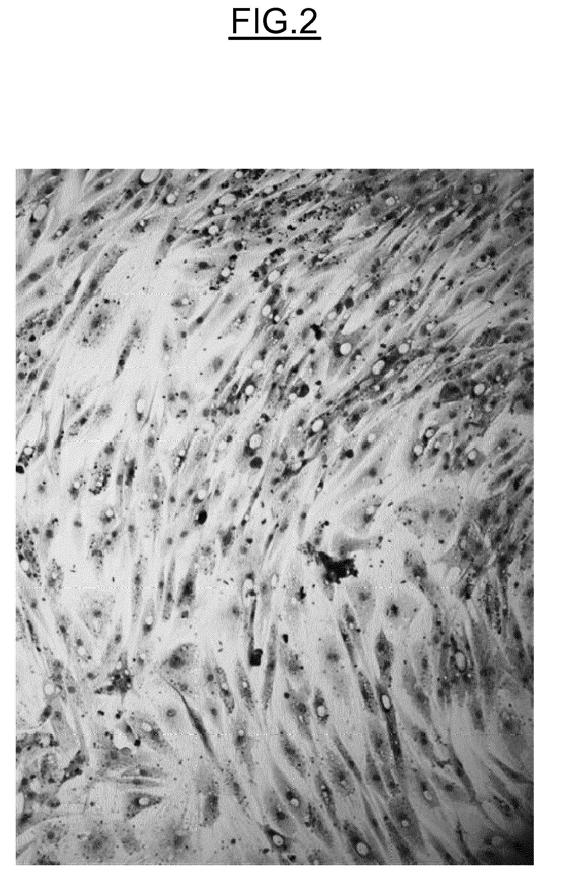

Des observations microscopiques (clichés photographiques FIG. 1 (témoin) et FIG. 2 (après traitement)) sont effectuées par un microscope plein champ inversé LEICA DMI6000B, statif inversé, platine motorisée XY, caméra LEICA DFC 300 mode couleur, objectif x40 (épaisseur lamelle variable) +DIC.

Nous observons un net changement de la morphologie des cellules avec les trois actifs après 3 jours (FIG. 2). En effet, les cellules ne contiennent plus de lipide (les vacuoles sont vides) et les cellules sont devenues polygonales avec un noyau central. De plus, des espaces intercellulaires apparaissent.

### c) Mesure du taux de glycérol sécrété

Des préadipocytes humains sont mis en culture à raison de 10 000 cellules / puits en plaques de 24 puits (Greiner) dans du milieu de croissance (Promocell®, réf : C-27410, France) incubés à 37°C dans une atmosphère de 5% CO₂. Lorsque les cellules sont sub-confluentes, (J0) elles sont incubées 3 jours en milieu de différenciation (Promocell ® C-24736), puis en milieu de nutrition (Promocell ® C-27438) pendant 13 jours. Puis on dépose 1,5 mL de la composition contenant les actifs dilués dans du milieu de nutrition sur les cellules pendant 72h.

Après traitement, les surnageants cellulaires sont retirés et stockés à -20°C avant dosage colorimétrique par le kit « Lipolysis Assay Kit » (ZenBio cat#LIP-1, Tebu-bio, Le Perray en Yvelines, France). La quantification se mesure par lecture spectrophotométrique à 540nm.

| Lecture à λ=540 nm | Quantité de glycérol (en µM) |
|---|---|
| Témoin | 63,9 |
| Apres traitement | 40,1 |

Ainsi, on constate que les cellules traitées par la composition contenant les trois actifs sécrètent moins de glycérol dans le milieu de culture que des cellules non traitées. Il y a en fait une réduction de 62% du taux de glycérol sécrété dans les cellules traitées par rapport aux cellules témoins non traitées.

### d) Etude de l'Expression de la protéine UCP1 par immunofluorescence

Cette protéine, exprimée dans les mitochondries, est considérée comme un des marqueurs spécifiques des adipocytes bruns.

Brièvement, les préadipocytes sont incubées une nuit (2x10⁴ cellules/puits en Lab-tek® 8 puits (Nunc)) dans du milieu de culture jusqu'à obtenir l'adhérence des cellules. Une fois les cellules adhérentes (J0), 300µL de milieu de différenciation est ajouté dans les puits pour les contrôles, et le milieu de différenciation additionné du mélange d'actif est ajouté pour le test. Après 6 jours, les cellules sont fixées pendant 15 minutes avec un excès de paraformaldéhyde dilué à 4% dans du PBS, puis perméabilisées par traitement pendant 3 minutes avec un excès de Triton dilué à 0.1% dans du PBS, les sites non spécifiques sont saturés par incubation durant une heure de PBS avec 3% BSA et 10% de Donkey Serum (Invitrogen USA). Puis les cellules sont marquées par l'anticorps Goat Ab primaire anti-UCP1 (Santa Cruz Biotechnology, Inc. USA) dilué au 1/200 en chambre humide à 4°C pendant la nuit, suivi du marquage par les anticorps secondaires couplés Alexa fluor 568 (donkey anti goat) fourni par Invitrogen USA, à la dilution de 1/200, pendant deux heures à température ambiante, en présence de réactif de Hoechst dilué au 1/10 000 pour l'identification des noyaux cellulaires.

La lecture des photos s'effectue à l'aide d'un microscope inversé à épifluorescence (Nikon, modèle TE 2000-E).

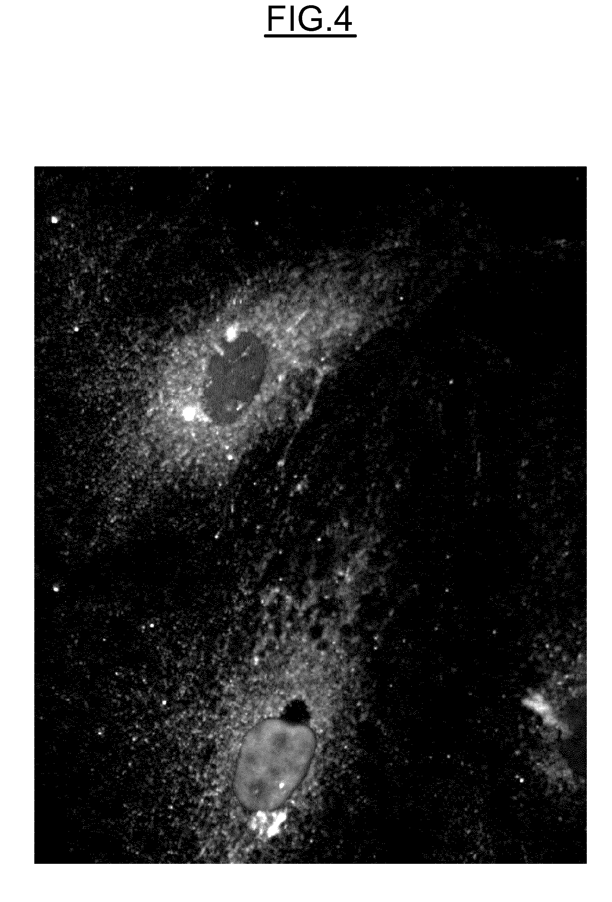

Dans ce test, les noyaux des cellules sont marqués en bleu (gris foncé sur les figures) et les protéines exprimant UCP 1 sont marquées en rouge (gris plus clair sur la figure 4). On peut noter qu'en présence de la composition contenant les actifs selon l'invention, les cellules expriment UCP1 (marquage gris plus clair sur la figure 4).

Les expériences montrent ainsi qu'après traitement avec la composition contenant les actifs de l'invention, on observe une réduction de 62% du taux de glycérol et une réduction parallèle du taux de triglycérides intracellulaires.

Ces résultats démontrent le pouvoir de cette combinaison spécifique d'actifs à induire *in vitro* un changement de phénotype des adipocytes blancs.

La modification du métabolisme cellulaire est indiscutable et l'apparition de nombreuses microvésicules lipidiques ainsi que l'expression de la protéine UCP-1 confirment l'induction d'une différenciation des adipocytes vers un phénotype de type brun.

### Exemple 4 : Formulation d'une crème huile dans eau (en %)

| Eau | QSP 100 |
|---|---|
| Squalane | 5.00 |
| Petrolatum | 5.00 |
| Glycérine | 5.00 |
| Isodecyl Neopentanoate | 5.00 |
| Pentaerythrityl Tetraethylhexanoate | 5.00 |
| Cyclomethicone | 4.00 |
| Cetearyl Alcohol | 3.00 |
| Myristyl Myristate | 2.00 |
| Laureth-23 | 2.00 |
| Silice | 2.00 |
| Cire d'abeille | 1.00 |
| Sclerotium Gomme | 1.00 |
| PEG-6 | 1.00 |
| Polyacrylamide | 0.80 |
| Glyceryl Stearate | 0.70 |
| Dimethiconol | 0.70 |
| Cetearyl Glucoside | 0.60 |
| C13-14 Isoparaffine | 0,40 |
| Acide Citrique | 0,14 |
| Laureth-7 | 0,10 |
| Caféine | 0.1 à 15 |
| Extrait de Saule Blanc | 0.01 à 10 |
| Extrait de Lotus | 0.01 à 10 |
| Pentapeptide-25 | 0,0001 à 5 |
| Preservative system | QS |
| Parfum | QS |

### Exemple 5 :Formulation d'une crème eau dans huile (en %)

| Eau | QSP 100 |
|---|---|
| Polyisobutène Hydrogéné | 7.00 |
| Isocetyl Stearate | 7.00 |
| Cyclométhicone | 4.80 |
| Glycérine | 4.00 |
| Huile Minérale | 3.00 |
| Oxyde de Zinc | 3.00 |
| Butylene Glycol | 2.00 |
| Isononyl Isononanoate | 2.00 |
| Cire d'abeille | 2.00 |
| Cetyl Dimethicone Copolyol | 1.70 |
| Polyglyceryl-4 Isostearate | 1.65 |
| Hexyl laurate | 1.65 |
| Disodium tartrate | 1.60 |
| Chlorure de Sodium | 1.00 |
| PEG-6 | 1.00 |
| 4,5,7-Trihydroxyisoflavone | 0,01 à 10 |
| Retinyl palmitate | 0.01 à 10 |
| Caféine | 0.1 à 15 |
| Extrait de Saule Blanc | 0.01 à 10 |
| Extrait de Lotus | 0.01 à 10 |
| Pentapeptide-25 | 0,0001 à 5 |
| Lentinus edodes | 0,02 à 5 |
| Preservative system | QS |
| Parfum | QS |

### Exemple 6 : Formulation d'un stick (en %)

| Huile de Castor | QSP 100 |
|---|---|
| Oleyl Alcohol | 20,00 |
| Hydrogenated Palm Kernel Oil | 17,00 |
| Cire de Candelilla | 11,00 |
| Polyglyceryl-3 Beeswax | 10,00 |
| Huile Minérale | 9,57 |
| Karité | 2,00 |
| Quaternium-18 Hectorite | 1,10 |
| Dioxyde de titane | 1,00 |
| Acétate de Tocophéryl | 0,50 |
| Carbonate de Propylène | 0,33 |
| Parfum | QS |
| Rétinol | 0.01 à 10 |
| Caféine | 0.1 à 15 |
| Extrait de Saule Blanc | 0.01 à 10 |
| Extrait de Lotus | 0.01 à 10 |
| Pentapeptide-25 | 0,0001 à 5 |

### Exemple 7 : Formulation d'un gel crème (en %)

| Eau | QSP 100 |
|---|---|
| Cyclométhicone | 5,40 |
| Octyl Palmitate | 5,00 |
| Hydrogenated Coco-glycérides | 3,00 |
| Arachidyl Behenyl Alcohol | 2,55 |
| Propylène Glycol | 2,50 |
| Isodecyl Neopentanoate | 2,00 |
| Glyceryl Stearate | 1,70 |
| Cetyl Alcohol | 1,30 |
| Acide Stéarique | 1,00 |
| PEG-6 | 1.00 |
| Cire d'abeille | 0,40 |
| C13-14 Isoparaffine | 0,40 |
| Butylene Glycol | 0,16 |
| Glycérine | 0,16 |
| Cetearyl Alcohol | 0,10 |
| Cetyl Palmitate | 0,10 |
| Cocoglycérides | 0,10 |
| Laureth-7 | 0,10 |
| Caféine | 0.1 à 15 |
| Extrait de Saule Blanc | 0.01 à 10 |
| Extrait de Lotus | 0.01 à 10 |
| Pentapeptide-25 | 0,0001 à 5 |
| Preservative system | QS |
| Parfum | QS |

### Exemple 8 : Formulation d'un spray (en %)

| Eau | QSP 100 |
|---|---|
| Glycérine | 4.00 |
| Montmorillonite | 3.00 |
| PEG-6 | 3.00 |
| Glycine | 0.30 |
| Acide Citrique | 0.09 |
| Caféine | 0.1 à 15 |
| Extrait de Saule Blanc | 0.01 à 10 |
| Extrait de Lotus | 0.01 à 10 |
| Pentapeptide-25 | 0,0001 à 5 |
| Preservative system | QS |
| Parfum | QS |

### Exemple 9 : Formulation d'une crème (exprimée en %)

| Eau | QSP 100 |
|---|---|
| Diméthicone | 3,00000 |
| Caféine | 1 à 15 |
| Glycérine | 2,00000 |
| Cyclopentasiloxane | 1,50000 |
| Butylène glycol | 1,00000 |
| Cyclohexasiloxane | 1,00000 |
| Polyacrylamide | 0,50000 |
| Parfum | QSP |
| C13-14 isoparaffine | 0,50000 |
| Sodium acrylates copolymère | 0,30000 |
| Polyisobutène hydrogéné | 0,20000 |
| Acrylates/C10-30 alkyl acrylate crosspolymer | 0,15000 |
| Trométhamine | 0,15000 |
| Laureth-7 | 0,12000 |
| Protéine de blé hydrolysée | 0,10000 |
| Lécithine | 0,10000 |
| Menthol | 0,10000 |
| Coco-glucoside | 0,07500 |
| Huile de graine d'Helianthus annuus (tournesol) | 0,06500 |
| Phospholipides | 0,06500 |
| Polyglyceryl-10 stéarate | 0,06500 |
| Caprylyl glycol | 0,03750 |
| Tocophérol | 0,03065 |
| PPG-1-PEG-9 Lauryl glycol éther | 0,00750 |
| Polyacrylate de sodium | 0,00700 |
| Silice | 0,00400 |
| Glaucine | 0,00250 |
| Décapeptide-2 | 0,00010 |
| Biotine | 0,00002 |
| Extrait de Saule Blanc | 0,01 à 10 |
| Extrait de Lotus | 0,01 à 10 |
| Pentapeptide-25 | 0,0001 à 5 |

## Revendications

1. Composition comprenant, dans un milieu physiologiquement acceptable, au moins un extrait de saule, un extrait de lotus et un peptide de séquence SEQ N°1.

2. Composition selon la revendication 1, dans laquelle l'extrait de saule est un extrait de saule blanc.

3. Composition selon la revendication 1 ou 2, dans laquelle l'extrait de saule blanc est un extrait hydroalcoolique.

4. Composition selon l'une quelconque des revendications 1 à 3, dans laquelle l'extrait de lotus est un extrait hydroalcoolique.

5. Composition selon l'une quelconque des revendications 1 à 4, dans laquelle la concentration finale d'extrait de saule blanc est de 0.01 à 10% en poids de la composition totale.

6. Composition selon l'une quelconque des revendications 1 à 5, dans laquelle la concentration finale d'extrait de lotus est de 0.01 à 10% en poids de la composition totale.

7. Composition selon l'une quelconque des revendications 1 à 6, dans laquelle la concentration de peptide de séquence SEQ ID N°1 est de 0,0001 à 5% en poids de la composition totale.

8. Composition selon l'une quelconque des revendications 1 à 7, **caractérisée par le fait qu'**elle comprend une phase aqueuse.

9. Composition selon la revendication 8, **caractérisée par le fait qu'**elle comprend de l'eau en une teneur allant de 1 à 99% en poids par rapport au poids total de la composition.

10. Composition selon la revendication 8, **caractérisée par le fait qu'**elle comprend de l'eau en une teneur allant de 40 à 75 % en poids par rapport au poids total de la composition.

11. Composition selon l'une quelconque des revendications 1 à 10, **caractérisée par le fait qu'**elle se présente sous la forme d'émulsion huile-dans-eau, de gel, de crème, ou sous la forme d'un stick.

12. Procédé cosmétique pour lutter contre la cellulite et/ou la peau d'orange, comprenant l'application topique sur la peau d'une composition selon l'une quelconque des revendications 1 à 11.


SEQUENCE LISTING

<110>  CASTER

<120>  Composition topique à visée amincissante

<130>  B11/1053FR GD/LM

<160>  1

<170>  PatentIn version 3.1

<210>  1
<211>  5
<212>  PRT
<213>  Artificial Sequence

<220>
<223>  fragment of UCP

<400>  1

Lys Val Arg Leu Gln
1               5